# EUROPÄISCHE PATENTSCHRIFT

(11) **EP 2 028 493 B1**
(45) Veröffentlichungstag und Bekanntmachung des Hinweises auf die Patenterteilung: **28.07.2010**
(21) Anmeldenummer: 08075749.5
(22) Anmeldetag: 13.10.1999
(51) Int. Cl.: G01N 33/74, C07K 14/585, A61K 38/23, A61K 38/22

(54) **Procalcitonin 3-116**
Procalcitonin 3-116
Procalcitonine 3-116

(30) Priorität: 15.10.1998 DE 19847690
(43) Veröffentlichungstag der Anmeldung: 25.02.2009
(62) Teilanmeldung aus: 06026319.1
(73) Patentinhaber: B.R.A.H.M.S Aktiengesellschaft, 16761 Hennigsdorf (DE)
(72) Erfinder: Bergmann, Andreas, 12351 Berlin (DE); Struck, Joachim, 13465 Berlin (DE); Weglöhner, Wolfgang, 12099 Berlin (DE)
(74) Vertreter: Kilger, Ute

(56) Entgegenhaltungen:
- ASSICOT M ET AL: "HIGH SERUM PROCALCITONIN CONCENTRATIONS IN PATIENTS WITH SEPSIS AND INFECTION" LANCET THE,GB,LANCET LIMITED. LONDON, Bd. 341, Nr. 8844, 27. Februar 1993 (1993-02-27), Seiten 515-518, XP000371780 ISSN: 0140-6736
- WRENGER, S. ET AL: "Amino-terminal truncation of procalcitonin, a marker for systemic bacterial infections, by dipeptidyl peptidase IV (DP IV)." FEBS LETTERS, VOL. 466, NO. 1, PP. 155-159., 21. Januar 2000 (2000-01-21), XP002143500

## Beschreibung

Die vorliegende Erfindung betrifft neue Diagnose möglichkeiten, die sich aus neuen, experimentell abgesicherten Erkenntnissen im Zusammenhang mit dem Auftreten von Procalcitonin-Teilpeptiden bei Sepsis und sepsisähnlichen schweren systemischen Infektionen ableiten ließen.

Aus den Patenten DE 42 27 454 bzw. EP 0 656 121 B1 bzw. US 5,639,617 ist es bekannt, daß die Bestimmung des Prohormons Procalcitonin bzw. von sich davon ableitenden Teilpeptiden in einem Serum oder Plasma eines Patienten, bei dem ein Sepsisrisiko besteht bzw. bei dem sepsistypische Krankheitserscheinungen festgestellt werden, ein wertvolles diagnostisches Hilfsmittel zur Früherkennung, d.h. zur Erkennung von Infektionen, die zu Sepsis führen können, und deren Abgrenzung von nicht-infektiösen Ätiologien, zur Erkennung des Schweregrads und zur therapiebegleitenden Verlaufsbeurteilung von Sepsis und sepsisähnlichen systemischen Infektionen darstellt. Besonders wertvoll hat sich die genannte Bestimmung zur differentialdiagnostischen Unterscheidung von Krankheitserscheinungen, die auf systemische mikrobielle Infektionen zurückzuführen sind, von anderen Krankheitserscheinungen nicht-infektiöser Ätiologie erwiesen, die aufgrund ihres klinischen Erscheinungsbildes auf eine Sepsis hindeuten könnten, jedoch in Wirklichkeit nicht auf eine systemische mikrobielle Infektion zurückzuführen sind, z.B. von Krankheitserscheinungen, die auf nicht-infektiöse Entzündungen einzelner Organe, auf postoperative Abstoßungsreaktionen oder Krebserkrankungen zurückzuführen sind. Auch können systemische Entzündungen von lokalen abgegrenzt werden.

Bezüglich eines Überblicks über den jüngeren Erkenntnisstand wird verwiesen auf W. Karzai et al. in Infection, Vol. 25 (1997), 6, S. 329-334 und die darin zitierte bzw. erwähnte weitere Fachliteratur.

Procalcitonin ist bekanntgeworden als Prohormon von Calcitonin, und seine vollständige Aminosäuresequenz ist seit längerem bekannt (FEBS 167 (1984), S. 93-97). Procalcitonin wird unter normalen Umständen in den C-Zellen der Schilddrüse produziert und dann spezifisch in das Hormon Calcitonin sowie die weiteren Teilpeptide Katacalcin und einen N-terminalen Rest aus 57 Aminosäuren ("Aminoprocalcitonin") gespalten.

Da bei Sepsis stark erhöhte Procalcitonin-Spiegel auch bei Patienten beobachtet werden, denen die Schilddrüse völlig entfernt wurde, mußte der Schluß gezogen werden, daß das im Blut von Sepsispatienten nachweisbare Procalcitonin außerhalb der Schilddrüse gebildet wird, wobei bezüglich der Organe bzw. Zellen oder der Gewebe, die für die Procalcitonin-Produktion bei Sepsis bestimmend sind, in der Fachliteratur unterschiedliche Vermutungen, die teilweise durch experimentelles Material gestützt wurden, geäußert wurden.

Was die Natur des bei Sepsis als "Procalcitonin" bestimmten Peptids angeht, so wurde zwar in den o.g. Patenten von Anfang an klargestellt, daß das bestimmte Peptid nicht völlig mit dem bekannten Procalcitonin-Peptid voller Länge identisch sein muß, das in den Schilddrüsen als Calcitonin-Vorläufer gebildet wird. Die Frage, ob sich das im Falle von Sepsis gebildete Procalcitonin von dem in den Schilddrüsen gebildeten Procalcitonin unterscheidet, blieb jedoch bisher ungeklärt. Als mögliche Unterschiede waren posttranslationale Modifikationen des bekannten Procalcitonins wie Glykosylierungen, Phosphorylierungen oder Veränderungen der Primärstruktur denkbar, jedoch auch modifizierte, verkürzte oder verlängerte Aminosäuresequenzen. Da die bisher angewandten analytischen Bestimmungsverfahren keine Unterschiede zwischen dem als Calcitonin-Vorläufer bekannten Procalcitonin und dem bei Sepsis gebildeten Procalcitonin erkennen ließen, wurde vorläufig allgemein davon ausgegangen, daß das bei Sepsis gebildete Procalcitonin mit dem Calcitonin-Vorläufer identisch ist und somit ein Peptid mit der bekannten Procalcitonin-Sequenz von 116 Aminosäuren (Procalcitonin 1-116) darstellt.

Wie die nachfolgend im experimentellen Teil dieser Anmeldung genauer erläuterten Bestimmungen im Labor der Anmelderin ergeben haben, unterscheidet sich das bei Sepsis gebildete Procalcitonin jedoch zwar geringfügig, jedoch signifikant von dem in der Schilddrüse gebildeten vollständigen Procalcitonin 1-116. Aus den festgestellten Unterschieden ergaben sich dann eine Reihe von wissenschaftlichen Schlußfolgerungen, die in neue diagnostische und therapeutische Verfahren, darin verwendbare Substanzen bzw. weiterverfolgbare wissenschaftliche Ansätze umgesetzt werden konnten.

Ausgangspunkt für den in der vorliegenden Patentanmeldung offenbarten Erfindungskomplexes ist die überraschende Erkenntnis, daß das bei Sepsis und sepsisähnlichen systemischen Infektionen im Serum von Patienten in vergleichsweise hohen Konzentrationen nachweisbare Procalcitonin nicht das vollständige Procalcitonin 1-116 mit 116 Aminosäuren ist, sondern ein am Aminoterminus um ein Dipeptid verkürztes, ansonsten jedoch identisches Procalcitonin mit einer Aminosäuresequenz von nur 114 Aminosäuren (Procalcitonin 3-116).

Das gegenüber dem vollständigen Procalcitonin fehlende Dipeptid weist die Struktur Ala-Pro auf. Das Fehlen eines Dipeptids mit einem Prolinrest als zweite Aminosäure des Amino-Terminus des vollständigen Procalcitoninsequenz gab Anlaß zu der Vermutung, daß bei der Erzeugung des bei Sepsis nachweisbaren Procalcitonins 3-116 möglicherweise eine bestimmte Peptidase eine Rolle spielt, und zwar die sogenannte Dipeptidyl-(Amino)-Peptidase IV (DP IV oder DAP IV bzw. CD26).

Zur Bestimmung einer möglichen Rolle der Dipeptidyl-Aminopeptidase IV im Rahmen von systemischen Infektionen bzw. von Sepsis haben die Erfinder daher experimentell geprüft, ob eine Korrelation der physiologischen DAP IV Konzentrationen mit dem Nachweis einer Sepsis möglich ist. Die erhaltenen Ergebnissen zeigten eine derartige Korrelation.

Aus den erhaltenen genaueren Ergebnissen wurde ferner eine Hypothese entwickelt, daß das Auftreten hoher Procalcitonin-Konzentrationen bei Sepsis und systemischen Infektionen möglicherweise keine isolierte Erscheinung ist, sondern daß in ähnlicher Weise auch erhöhte Konzentrationen anderer Prohormone meßbar sein könnten, so daß die Bestimmung derartiger Prohormone eine mögliche Alternative zur Procalcitonin-Bestimmung darstellt bzw. geeignet ist, die Procalcitonin-Bestimmung in Einzelfällen zu ergänzen oder in diagnostisch signifikanter Weise weiter abzusichern.

Die Erkenntnis, daß bei Sepsis nicht das vollständige Procalcitonin 1-116 im Serum von Patienten gefunden wird, sondern ein verkürztes Procalcitonin 3-116, ist schließlich auch von potentiellem Interesse für die Sepsis-Therapie. In einem Artikel von Eric S Nylen et al., Crit Care Med 1998, Vol. 26, No. 6, S. 1001-1006 werden experimentelle Befunde beschrieben, die darauf hindeuten, daß das bei Sepsis auftretende Procalcitonin nicht nur ein diagnostisch wichtiger Marker ist, der z.B. als Stoffwechsel-Abfallprodukt entstanden ist, sondern in einem infektiös bedingten Entzündungsgeschehen eine aktive Rolle als Mediator zu spielen scheint, indem Procalcitonin eine Entzündungsreaktion aufrechterhalten und verstärken kann. Diese Rolle von Procalcitonin wird gegenwärtig kontrovers diskutiert, und die bekannt gewordenen Versuchsergebnisse ergeben kein einheitliches Bild.

Die o.g. Erkenntnis, daß bei Sepsis ein am Aminoterminus um zwei Aminosäuren verkürztes Procalcitonin auftritt, legt nunmehr die Vermutung nahe, daß das Procalcitonin, das im Falle von Sepsis und anderen entzündlichen systemischen Infektionen eine aktive Rolle spielt, dieses verkürzte Procalcitonin 3-116 sein dürfte, und daß Untersuchungen, die mit dem Procalcitoninpeptid voller Länge durchgeführt wurden, u.a. aus diesem Grunde abweichende oder widersprüchliche Ergebnisse lieferten. Es ist durchaus bekannt, daß viele physiologisch aktive Peptide durch Spaltung, z.B. eine einleitende Abspaltung eines kurzen Peptidrests, in ihre eingentliche aktive Form überführt werden. Ein bekanntes Beispiel ist Angiotensin, bei dem aus dem nichtaktiven Angiotensinogen mit 14 Aminosäuren durch sukzessive Abspaltung erst eines Tetrapeptids und dann eines Dipeptids sowie schließlich einer einzelnen Aminosäure Peptide mit erheblich unterschiedlichen physiologischen Aktivitäten gebildet werden. Daß relativ geringfügige Modifikationen am N-Terminus von physiologisch aktiven Peptiden im Rahmen des Immungeschehens eine Rolle spielen und zu erheblichen Aktivitätsveränderungen bei den entsprechenden Peptiden führen können, wird durch eine Reihe von Veröffentlichungen aus der jüngsten Zeit unterstrichen, in denen jedoch keine Beziehung zu septischen Krankheitsgeschehen hergestellt wird (vgl. z.B. J Immunol 1998, Sept. 15, 161(6):2672-5; Biochemistry 1998, Sept. 8, 37(36): 12672-80; FEBS Lett 1998, Juli 31, 432 (1-2):73-6; J Biol Chem 1998, März 27; 273 (13):7222-7; J Exp Med 1997, Dec 1; 186(11):1865-72).

Nimmt man an, daß Procalcitonin 3-116 aktiv an einem Entzündungsgeschehen beteiligt ist und für dieses verkürzte Procalcitonin spezifische molekulare Rezeptoren oder ähnliche spezifische Binder existieren, eröffnen sich neue therapeutische Möglichkeiten zur Beeinflussung des Verlaufs einer Sepsis unter Einsatz des Procalcitonins 3-116 bzw. von Agonisten und Antagonisten, die mit den Rezeptoren für das Procalcitonin 3-116 wechselwirken und damit die von diesem ausgelösten physiologischen Reaktionen und damit auch ein Entzündungsgeschehen beeinflussen können. Auch der Einsatz von spezifischen Bindern von Procalcitonin 3-116, z.B. selektiven Antikörpern, stellt einen therapeutischen Ansatz dar, der durch die hierin vermittelten Erkenntnisse eröffnet wird.

Schließlich ergab sich daraus, daß die Dipeptidyl-Aminopeptidase IV bei der Generierung des Procalcitonins 3-116 bei Sepsis und systemischen Infektionen eine Rolle spielen könnte, eine weitere Hypothese, nämlich daß eine Sepsis oder ein sepsisähnliches Entzündungsgeschehen möglicherweise auch dadurch therapeutisch beeinflußt werden könnte, daß man die Dipeptidyl-Aminopeptidase IV in ihrer Wirkung beeinflußt, indem man sie z.B. durch geeignete selektive Binder, Antikörper oder ähnliche Rezeptoren-Moleküle blockiert.

Es ist Ziel der vorliegenden Patentanmeldung, die sich aus den obigen neuen Erkenntnissen und daraus abgeleiteten Schlußfolgerungen ergebenden neuen technischen Lehren patentrechtlich abzusichern, soweit diese unter Berücksichtigung des derzeitigen Erkenntnisstandes einem Patentschutz zugänglich sind.
In ihrer breitesten Form ist die Erfindung im unabhängigen Anspruch 1 definiert. Bevorzugte Ausführungsformen finden sich in den abhängigen Ansprüchen 2 und 3:
   1. Verfahren zur Diagnose von Sepsis und sepsisähnlichen systemischen Infektionen, wobei Procalcitonin 3-116 in einer Probe aus dem Blut eines Patienten detektiert wird.
   2. Verfahren nach Anspruch 1, wobei die Probe aus dem Blut eine Serumprobe ist.
   3. Verfahren nach Anspruch 1, wobei die Probe aus dem Blut eine Plasmaprobe ist.

Nachfolgend wird unter Bezugnahme auf mehrere Diagramme ausgewähltes experimentelles Material vorgelegt, das die neuen Erkenntnisse untermauert bzw. das für die Richtigkeit der daraus abgeleiteten Annahmen spricht.

In den Figuren zeigen:
- Figur 1: die Ergebnisse einer Procalcitonin-Isolierung und Aufreinigung per HPLC aus einem Mischserum aus gesammelten Seren von verschiedenen Patienten mit schwerer Sepsis;
- Figur 2: die Ergebnisse einer massenspektroskopischen Un- tersuchung derjenigen Fraktionen des Mischserums aus Fig.1, die eine hohe Procalcitonin-Immunreak- tivität aufwiesen;
- Figur 3: Ergebnisse der Bestimmung der Enzymaktivität von Dipeptidyl-Aminopeptidase IV in septischen Seren und Normalseren; sowie
- Figur 4: die Ergebnisse der Bestimmung von Procalcitonin in septischen Seren und Normalseren in Gegenüberstel- lung mit Ergebnissen der Bestimmung eines weiteren Prohormons, nämlich pro-Gastrin-Releasing-Peptide (proGRP), in den gleichen Seren.
- Figur 5: die Ergebnisse der Bestimmung von Procalcitonin in
- Figur 6: Seren eines Kollektivs von 20 Normalpersonen sowie andererseits 20 Sepsispatienten. die Ergebnisse der Bestimmung von Pro-ANF (in pg/Röhrchen) in den gleichen Kollektiven von Nor- malpersonen und Sepsispatienten wie in Figur 5.
- Figur 7: die Ergebnisse der Bestimmung von Pro-ADM (in pg/Röhrchen) in den gleichen Kollektiven von Nor- malpersonen und Sepsispatienten wie in Figur 5.
- Figur 8: die Ergebnisse der Bestimmung von Pro-END (in pg/Röhrchen) in den gleichen Kollektiven von Nor- malpersonen und Sepsispatienten wie in Figur 5.
- Figur 9: die Ergebnisse der Bestimmung von Pro-BNP (in pg/Röhrchen) in den gleichen Kollektiven von Nor- malpersonen und Sepsispatienten wie in Figur 5.

### EXPERIMENTELLER TEIL

### A. Isolierung und Charakterisierung des endogenen Procalcitonin-Peptids aus Seren septischer Patienten

Durch Vermischen von Serumproben von verschiedenen Patienten mit schwerer Sepsis wurde ein Mischserum mit einem Gesamtvolumen von 68 ml hergestellt. Die Procalcitonin-Konzentration in dem erhaltenen Mischserum wurde mit Hilfe eines kommerziellen Procalcitonin-Assays (LUMItest PCT, B.R.A.H.M.S Diagnostica) zu 280 ng/ml (Gesamtmenge 19µg) ermittelt. Das Mischserum wurde mit einem gleichen Volumen eines Puffers (68 ml; 10mM EDTA, 1 mg/ml Maus-IgG, 2 mg/ml Schaf-IgG, 2 mg/ml bovines IgG, 0,1 mMol Leupeptin, 50 µM Amastatin in PBS) vermischt, und das in der Probe enthaltene Procalcitonin wurde affinitätschromatographisch isoliert und gereinigt.

Hierzu wurde die gesamte Mischprobe bei einer Durchflußrate von 0,5 ml/min viermal hintereinander über eine Affinitätssäule (0,5 x 1 cm, Anti-Calcitonin-Antikörper, gebunden an Carbolink der Firma Pierce, Procalcitonin-Bindekapazität ca. 20 µg) gepumpt. Anschließend wurde die Säule mit 30 ml PBS gewaschen, und das gebundene Peptid wurde mit Hilfe von 50 mM Essigsäure (pH ca. 2,0) eluiert. Der Säulenausfluß wurde kontinuierlich auf Absorption bei 280 nm überwacht, und die von der Essigsäure eluierte Proteinfraktion wurde aufgefangen (Endvolumen 2,0 ml).

Das auf diese Weise gesammelte Material wurde über Umkehrphasen-HPLC an einer rpC₁₈-Säule µ Bondapak 0,4 x 30 mm (Firma Waters) gereinigt. Die Durchflußgeschwindigkeit betrug 1 ml/min, Laufmittel und Elutionsbedingungen sind der folgenden Tabelle 1 zu entnehmen.

**Tabelle 1: Elutionsbedingungen rp-HPLC Procalcitonin**

| | | | |
|---|---|---|---|
| Laufmittel A: | 5 % Acetonitril | | |
| | 20 mM NH₄-Acetat | | |
| Laufmittel B: | 90 % Acetonitril | | |
| | 20 mM NH₄-Acetat | | |
| Gradient: | 0,0 min | 100 % A | 0 % B |
| | 2,5 min | 100 % A | 0 % B |
| | 5,0 min | 89 % A | 11 % B |
| | 55,0 min | 56 % A | 44 % B |
| | 60,0 min | 0 % A | 100 % B |

Der Säulenausfluß wurde kontinuierlich auf seine Absorption bei 214 nm vermessen und in Fraktionen von 0,25 ml gesammelt. Mit Hilfe eines handelsübliche Procalcitonin-Assays (LUMItest PCT, B.R.A.H.M.S Diagnostica) wurden diejenigen Fraktionen bestimmt, in denen eine PCT-Immunreaktivität nachweisbar war. Es zeigte sich, daß die Haupt-Immunreaktivität in der 51. Fraktion als scharfe Bande eluiert wurde. Außerdem wurden heterogen zusammengesetzte Proteinfraktionen mit geringeren PCT-Immunreaktivitäten in den Fraktionen 39 bis 49 erhalten.

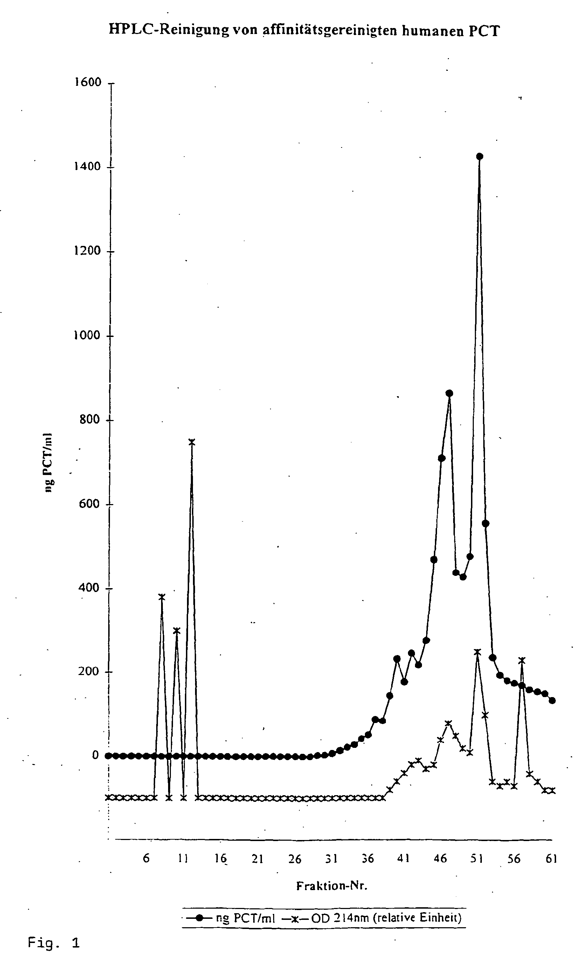

Figur 1 zeigt die ermittelte PCT-Immunreaktivität (ausgedrückt als ng PCT/ml) für die einzelnen gesammelten Fraktionen der rp HPLC-Chromatographie, überlagert einer Kurve, die die optische Dichte (OD) der eluierten Fraktionen darstellt.

Alle Fraktionen, die eine positive Procalcitonin-Immunreaktivität aufwiesen, wurden durch Begasen mit Stickstoff getrocknet. Anschließend wurden die Proben massenspektrometrisch analysiert und einer N-terminalen Sequenzierung unterworfen.

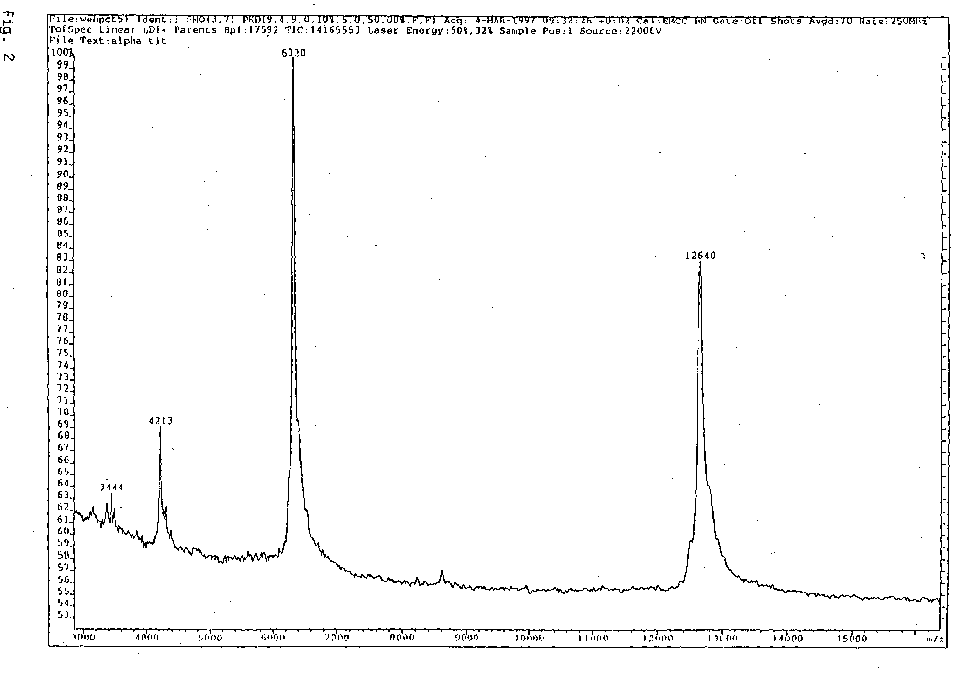

Bei der massenspektrometrischen Untersuchung (MALDI-TOF-Methode) wurde für die Fraktionen 50-52 das in Figur 2 gezeigte Profil erhalten, aus dem sich eine Molmasse von 12640 ±15 ergab. Sämtliche anderen massenspektrometrisch untersuchten Fraktionen (36-49, 53-59) ergaben heterogene Massenverteilungen mit Molmassen <12640. Ihre Einzelmasse ergab im Vergleich zur Intensität der Masse der Fraktion 50-52 eine Intensität von <2 %. Damit wurde nachgewiesen, daß die Procalcitonin-Immunreaktivität in Seren von Sepsispatienten mit einer Masse von 12640 ±15 assoziiert ist. Keine der gewonnenen Fraktionen wies eine höhere Masse auf.

Die in den Fraktionen 36-59 enthaltenen Peptide wurden einer N-terminalen Sequenzierung unterworfen. Auch hierbei erwies sich der Inhalt der Fraktionen 36-49 und 53-59 als heterogen, d.h. es wurde eine Vielzahl von unterschiedlichen N-Termini ermittelt.

Für die Fraktion 50-52, in denen die überwiegende Procalcitonin-Immunreaktivität zu finden war, ergab sich, daß die darin enthaltenen Peptide eindeutig folgenden N-Terminus (15 Aminosäuren) aufwiesen:
Phe Arg Ser Ala Leu Glu Ser Ser Pro Ala Asp Pro Ala Thr Leu

Das Peptid aus den Fraktionen 50-52 wurde anschließend mittels Protease Glu-C bzw. Trypsin verdaut, die entstandenen Fragmente wurden auf an sich bekannte Weise über SMART-HPLC gewonnen und anschließend massenspektrometrisch und sequenzanalytisch untersucht.

Es wurde eine Sequenz erhalten, die vollständig mit der Sequenz der Aminosäuren 3-116 des bekannten Procalcitonins 1-116 übereinstimmte. Die theoretische Masse dieser Sequenz betrug 12627, was mit der massenspektrometrisch ermittelten Masse von 12640±15 übereinstimmt.

Damit wurde nachgewiesen, daß im Blut von Sepsispatienten ein Procalcitonin-Peptid zirkuliert, das 114 Aminosäuren umfaßt und als Procalcitonin 3-116 zu bezeichnen ist. Das Peptid ist nicht durch posttranslationale Modifikationen wie Phosphorylierungen oder Glykosylierungen verändert.

Das Procalcitonin 3-116 wurde bisher in der wissenschaftlichen Literatur noch nicht als mögliches endogenes Procalcitonin-Teilpeptid diskutiert, und es bestand daher bisher für den Fachmann auch noch keinerlei Veranlassung, dieses Peptid gezielt herzustellen und auf seine Eigenschaften zu untersuchen. Die obigen Befunde lieferten nunmehr jedoch einen Anlaß zur gezielten Herstellung des genannten Procalcitonin 3-116 nach gentechnologischen Techniken. Seine Herstellung wird nachfolgend beschrieben.

### B. Klonierung, Expression und Aufreinigung von rekombinantem Procalcitonin 3-116.

### 1. Klonierung

Die für Procalcitonin 3-116 (nachfolgend abgekürzt PCT 114) codierenden DNA-Fragmente wurden unter Anwendung einer PCR-Amplifikation mit Hilfe geeigneter oligonukleotidprimer aus einem humanen Schilddrüsen-cDNA-Pool isoliert. Das gewünschte Fragmente wurde mittels gängiger Methoden (Ausubel FM, Brent R, Kingston RE, Moore DD, Seidman JG, Smith JA, und Struhl K (1991), Current Protocols in Molecular Biology, John Wiley & Sons, New York) kloniert, und die korrekte Nukleotidsequenz wurde durch DNA-Sequenzierung und Vergleich mit der bekannten, für Procalcitonin codierenden DNA-Sequenz verifiziert.

Für die Expression der für PCT 114 codierenden cDNA wurde ein Vektor verwendet, der neben einem T7-Promotor eine Region enthält, die für das Signalpeptid des sog. pelB-Proteins codiert. Dieses pelB-Signalpeptid sorgt dafür, daß ein nach Klonierung und Expression entstandenes Fusionsprotein durch die cytoplasmatische Membran der für die Expression verwendeten Wirtzellen in den periplasmatischen Raum transportiert wird. Bei diesem Transportvorgang wird gleichzeitig das N-terminale Signalpeptid durch eine membranständige Signalpeptidase abgetrennt (Stader JA und Silhavy TJ (1990), Engineering Escherichia coli to secrete heterologous gene products, Methods Enzymol. 185, 166-187). Diese Vorgehensweise garantiert, daß das gefundene Expressionsprodukt exakt die gewünschte Sequenz aufweist. Bei dieser Vorgehensweise fehlt das bei anderen Methoden zur Expression notwendige N-terminale Methionin.

Nach der Klonierung der cDNA für PCT 114 in einen Vektor der genannten Art und der Transformierung von E. coli mit diesem Vektor wurde Procalcitonin 3-116 exprimiert. Die periplasmatische Fraktion mit dem exprimierten Procalcitonin 3-116 wurde auf an sich bekannte Weise isoliert (Neu HC und Heppel LA (1965), The release of enzymes from Escherichia coli by osmotic shock and during the formation of spheroblasts, J. Biol. Chem. 240, 3685-3692). Nach einer Zentrifugierung (100.000 g, 30 min, 4°C) und Filtration des flüssigen Überstands (0,2 µm) wurde das erhaltene Filtrat durch Anionen-Austauschchromatographie aufgetrennt. Die Fraktionen mit Procalcitonin-Immunreaktivität wurden vereinigt und über Umkehrphasen-HPLC aufgereinigt, wie im Zusammenhang mit der Isolierung von PCT 3-116 aus septischen Seren beschrieben wurde.

Alle Fraktionen mit Procalcitonin-Immunreaktivität wurden vereinigt und lyophilisiert. Wie eine Überprüfung des Materials mittels SDS-PAGE ergab, war das so gewonnene Material zu mindestens 95% sauber.

Die Identität des exprimierten und aufgereinigten Peptids als Procalcitonin 3-116 wurde durch Massenspektrometrie und Sequenzanalyse bestätigt.

Das erhaltene rekombinante Procalcitonin 3-116 stellt ein neues rekombinantes Peptid dar und kann in dieser Form zur Herstellung von Immunreagenzien verwendet sowie auf seine Eignung als Therapeutikum bzw. auf seiner Fähigkeit, im Sinne der o.g. Veröffentlichung (Eric S Nylen, a.a.O.) prophylaktisch und therapeutisch zu wirken, untersucht werden.

Zur Herstellung von Kalibratoren für PCT-Assays wurde das oben für die gentechnologische Herstellung von Procalcitonin 3-116 beschriebene Verfahren in im wesentlichen identischer Form auch zur Herstellung des vollständigen Procalcitonins 1-116 und des Procalcitonins 2-116 eingesetzt.

### C. Ermittlung der Dipeptidyl-Aminopeptidase IV (DAP IV)-Aktivität in humanen Normalseren bzw. Seren von Patienten mit schwerer Sepsis

Jeweils 20 Serumproben von gesunden Normalpersonen sowie von Sepsispatienten wurden auf ihre für Dipeptidyl-Aminopeptidase IV-spezifische Enzymaktivität untersucht. Die DAP IV-Enzymaktivität wurde auf an sich bekannte Weise fluorometrisch unter Verwendung von Lys-Pro-4-Methoxy-beta-naphthyl-amid vermessen. Dazu wurden jeweils 2 µl des zu überprüfenden Serums mit 3 ml Substrat (50 µg/ml Lys-Pro-4-Methoxy-beta-naphylamid, 50 mM Tris/HCl, pH 7,5) vermischt, und die auftretende Fluoreszenz wurde kontinuierlich unter Anregung mit Licht einer Wellenlänge von 340 nm bei einer Emissionswellenlänge von 410 nm gemessen. Das Fluoreszenzsignal wurde mittels einer 4-Methoxy-naphthylamin-Lösung kalibriert. Die auf diese Weise ermittelte Enzymaktivität wird in nmol/- min angegeben.

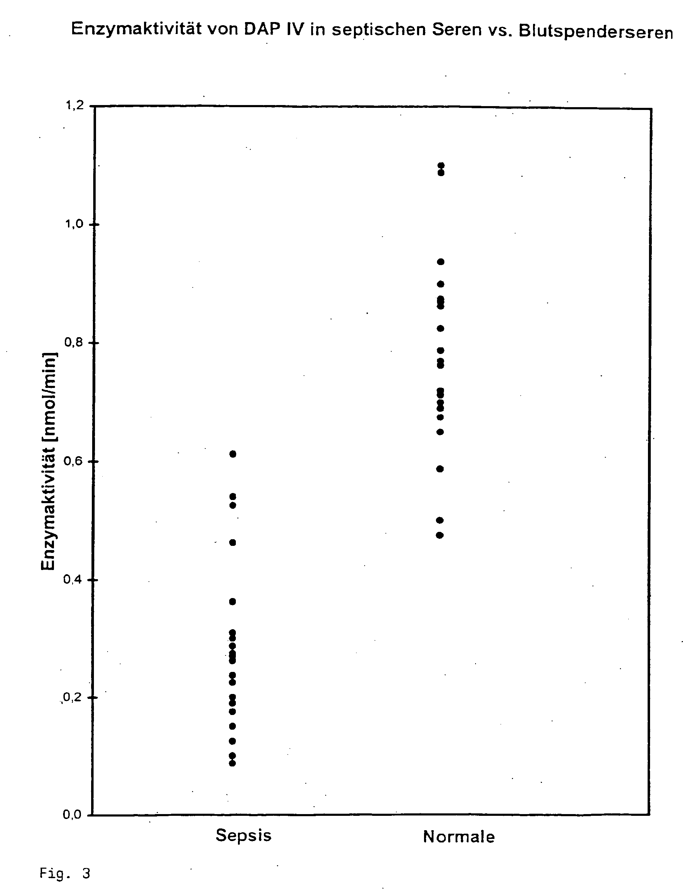

Die erhaltenen Ergebnisse sind Figur 3 dargestellt.

Es ist klar zu erkennen, daß die DAP IV-Enzymaktivität in den Sepsis-Seren deutlich niedriger liegt als in den Seren von gesunden Normalpersonen (Blutspenderseren). Damit läßt sich die Bestimmung der DAP IV-Enzymaktivität im Plasma oder Serum auch zur Erkennung von Sepsis in Patientenseren heranziehen.

Die bei Sepsis deutlich erniedrigte Plasmakonzentration an DAP IV kann einerseits als Beleg dafür angesehen werden, daß DAP IV an einem Sepsis-Geschehen mitbeteiligt ist. Andererseits deuten die Ergebnisse darauf hin, daß es nicht die Konzentration an DAP IV im Plasma sein kann, die für die Bildung von Procalcitonin 3-116 verantwortlich ist. Die erhaltenen Ergebnisse legen vielmehr den Schluß nahe, daß Procalcitonin 3-116 durch gewebs- bzw. zellgebundene DAP IV, möglicherweise intrazellulär, gebildet und aus Procalcitonin-erzeugenden oder speichernden Zellen freigesetzt wird.

Die der Literatur entnehmbare Information, daß DAP IV von aktivierten T-Zellen exprimiert wird, (vergleiche Hegen und Oravecz, Protein-Reviews on the WEB; Fleischer, a.a.O.) deutet auf einen engen Zusammenhang zwischen der Expression von DAP IV und dem Aktivierungszustand des Immunsystems hin, das bei einer septischen systemischen Infektion extrem belastet ist und daher typische Reaktionen zeigt, die sich u.a. in einer stark erhöhten Procalcitonin 3-116 Bildung äußern.

Abgesehen von den sich aus den obigen Befunden ergebenden Möglichkeiten, DAP IV im Rahmen der Sepsis-Diagnose zu bestimmen, können die obigen Ergebnisse auch darauf hindeuten, daß die bei einer Sepsis kaskadenartig ablaufenden Vorgänge durch DAP IV-Hemmstoffe therapeutisch beeinflußt werden können, wodurch es möglich sein könnte, die Freisetzung von Procalcitonin 3-116 sowie anderen Hormonen oder konvertierten Prohormonen unter Sepsis zu verhindern oder zu vermindern, so daß pathologische Konsequenzen dieser Prohormonfreisetzung vermindert oder vermieden werden können.

### D. Bestimmung der Konzentrationen anderer Prohormone als Procalcitonin bei Sepsis

Die Tatsache, daß bei Sepsis nicht das vollständige Prohormon Procalcitonin, sondern ein modifiziertes, um ein Xaa-Pro-Dipeptid verkürzte Prohormon freigesetzt wird, führte zu der Hypothese, daß bei Sepsis nicht nur Procalcitonin 3-116 freigesetzt wird, sondern daß Procalcitonin 3-116 vielleicht nur ein Vertreter aus einer ganzen Gruppe von Prohormonen oder ähnlichen Peptiden, z.B. solchen mit immunmodulatorischen Eigenschaften, ist, die bei Sepsis in erhöhtem Maße und möglicherweise konvertierter Form freigesetzt werden.

Eine Überprüfung bekannter Prohormone und der für diese Prohormone der Literatur entnehmbaren Aminosäuresequenzen zeigte, daß tatsächlich bei den meisten bekannten Prohormonen am Aminoterminus ein Dipeptid zu finden ist, das als Xaa-Pro definiert werden kann und das daher in einem ähnlichen Sinne, wie beim Procalcitonin beobachtet, abgespalten werden kann. Im einzelnen finden sich am Aminoterminus sehr vieler Prohormone oder Immunmodulatoren Dipeptide des genannten Typs. Die nachfolgende tabellarische Übersicht über Literaturdaten gibt einen Überblick über einige ausgewählte Prohormone bzw. Immunmodulatoren, das bei diesen am Amino-Terminus zu findende Dipeptid sowie ihre Gesamtzahl der Aminosäuren.

Die in der Tabelle 2 aufgeführten Prohormone stellen Beispiele für Prohormone dar, deren Konzentrationen bei Sepsis erhöht sein können, ohne daß die Aufzählung als erschöpfend anzusehen ist. Bei den Immunmodulatoren ist mit einer Beeinflussung der Aktivität durch Abspaltung eines Dipeptids zu rechnen.

**Tabelle 2:**

| Pro-Hormon/ Immunmodulator | Dipeptid am N-Terminus | Gesamtzahl aller Aminosäuren |
|---|---|---|
| pro-Endothelin-1 (pro-END) | Ala Pro | 195 |
| pro-Brain-Natriuretic-Peptide (pro-BNP) | His Pro | 108 |
| pro-Atrial-Natriuretic-Peptide (pro-ANP; auch pro-Atrionatriuretischer Faktor, pro-ANF) | Asn Pro | 128 |
| pro-Leptin | Val Pro | 146 |
| pro-Neuropeptide Y | Tyr Pro | 69 |
| pro-Somatostatin | Ala Pro | 92 |
| pro-Neuropeptide YY | Thr Pro | 69 |
| Interleukin 6 | Val Pro | 183 |
| Interleukin 10 | Ser Pro | 160 |
| pro-Gastrin-Releasing Peptid (proGRP) | Val Pro | 115 |
| pro-Opiomelanocortin | Trp Cys | 241 |
| pro-Adrenomedullin (pro-ADM) | Ala Arg | 164 |
| Procalcitonin (PCT) | Ala Pro | 116 |

Die bisherigen experimentellen Befunde deuten tatsächlich darauf hin, daß bei einer systemischen Infektion wie Sepsis generell Prohormone und/oder Peptid-Immunmodulatoren wie Interleukine, möglicherweise unter Modifizierung durch Abspaltung von Dipeptiden am Amino-Terminus, freigesetzt werden und daß diese durch Wechselwirkung mit zugehörigen spezifischen Rezeptoren oder anderen Bindern möglicherweise weitere Folgeschritte in der Kaskade einer Immunantwort auslösen.

Es wurde parallel zu einer Procalcitonin-Bestimmung in Normalseren und Seren von Sepsis-Patienten auch die Bestimmung weiterer Prohormone durchgeführt, die eher willkürlich ausgewählt ausgewählt worden waren. Es waren dies (i) das pro-Gastrin-Releasing Peptide (proGRP), (ii) das pro-Atrial-Natriuretic-Peptide (pro-ANP bzw. pro-ANF), (iii) das pro-Adrenomedullin (pro-ADM), (iv) das pro-Endothelin (pro-END) und (v) das pro-Brain-Natriuretic-Peptide (pro-BNP).

### D.1. Bestimmung von proGRP in Seren von Sepsis-Patienten und von Normalpersonen

Für die Bestimmung von proGRP ist ein Assay im Handel. ProGRP wird in einer jüngeren Veröffentlichung als Tumormarker beim kleinzelligen Bronchialkarzinom beschrieben (Petra Stieber et al, J Lab Med 1997, 21(6):336-344). Der Assay zur Bestimmung von ProGRP wird von der Tonen Corporation unter der Bezeichnung ProGRP ELISA KIT^{™} im Handel angeboten.

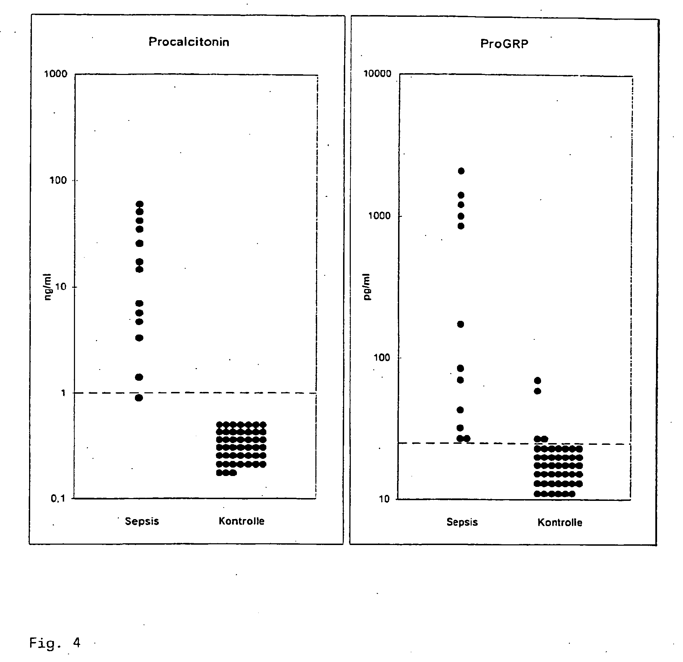

Unter Verwendung dieses Kits wurden unter Einhaltung der Arbeitsweise, die in der Gebrauchsinformation des kommerziellen Kits vorgeschrieben ist, die in Figur 4 dargestellten Meßergebnisse erhalten.

Ein Vergleich der für Procalcitonin einerseits und ProGRP andererseits erhaltenen Werte zeigt, daß bei Procalcitonin die Unterscheidung zwischen Normalseren und Sepsis-Seren zwar eindeutiger ist, daß aber die ProGRP-Konzentrationen in weitgehend ähnlicher Weise erhöht sind wie die von Procalcitonin.

### D.2. Bestimmung von pro-ANF, pro-ADM, pro-END und pro-BNP in Seren von Sepsis-Patienten und von Normalpersonen

Zur Bestimmung der (Prä-)Prohormone pro-ANF, pro-ADM, pro-END bzw. pro-BNP sind Assays der Firma DRG (DRG Instruments GmbH, D-35018 Marburg, Deutschland) in Kitform im Handel erhältlich, die für die nachfolgenden Messungen unter Beachtung der Anweisungen der Hersteller verwendet wurden.

### Im einzelnen wurden verwendet:

Zur Bestimmung von pro-ANF der Prepro-ANF 26-55 (human) RIA Kit, zur Bestimmung von pro-ADM der Pro-Adrenomedullin 45-92 (human) RIA Kit, zur Bestimmung von pro-END der Prepro-Endothelin 18-50 (human) RIA Kit, und zur Bestimmung von Pro-BNP der Prepro-BNP 22-46 (human) RIA Kit der o.g. Firma DRG.

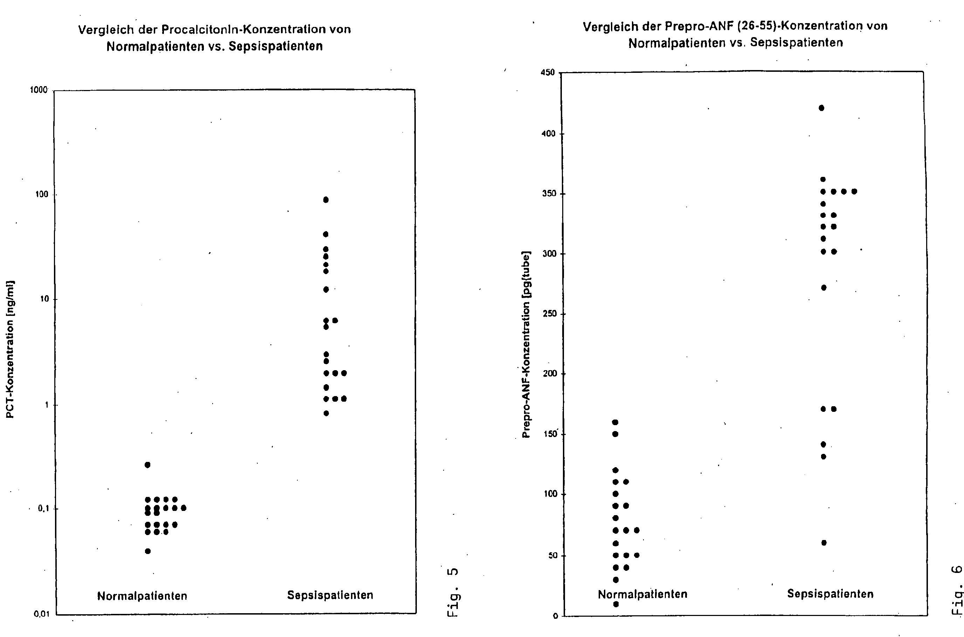

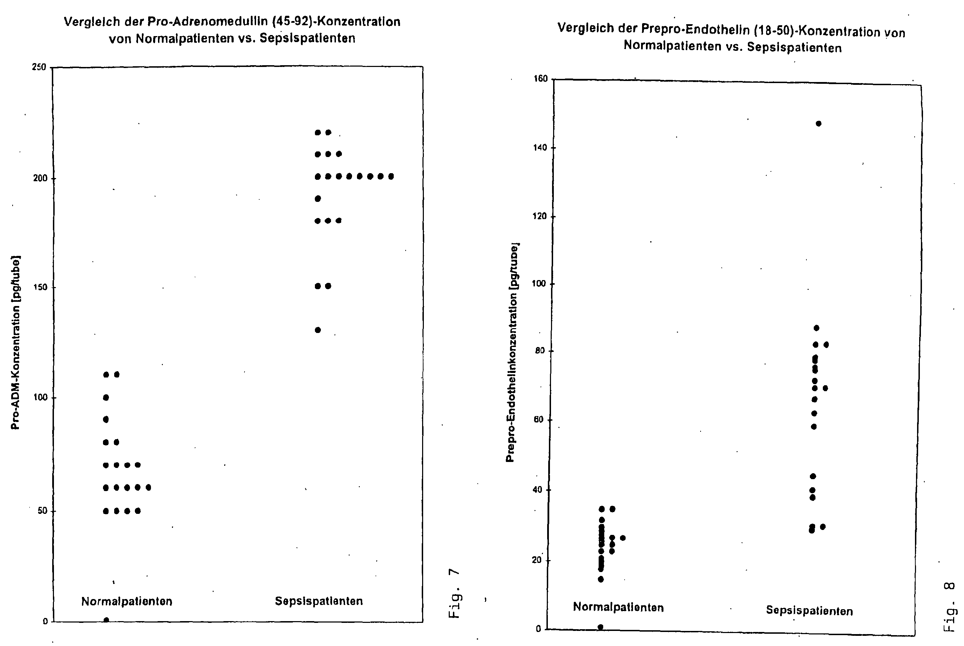

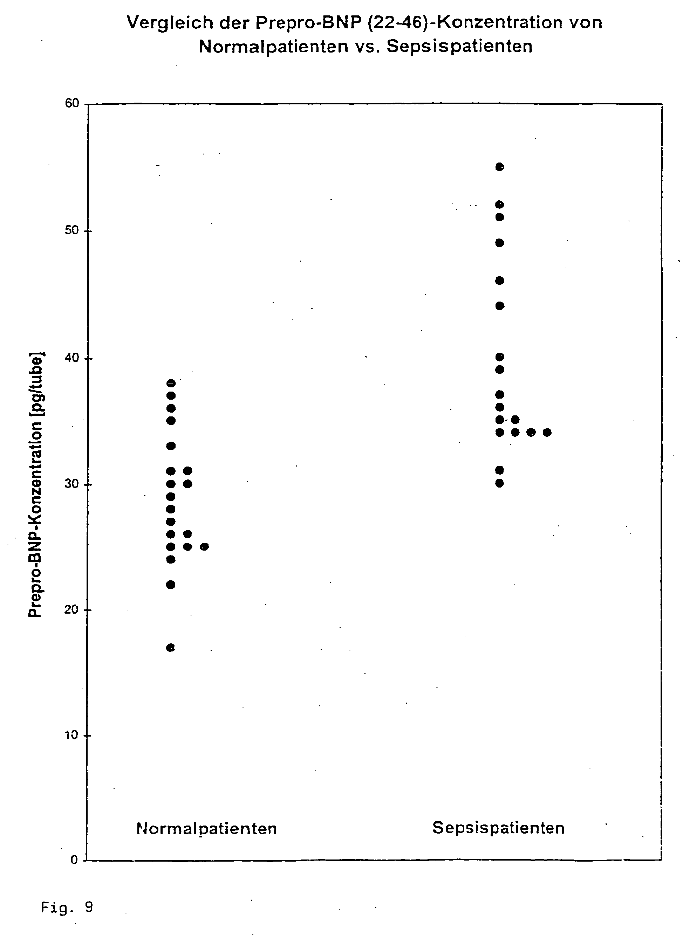

In den Seren eines Kollektivs von 20 Normalpersonen A-T sowie von 20 Sepsispatienten wurden die o.g. Prohormone sowie, parallel dazu, Procalcitonin bestimmt. Die Ergebnisse sind in der nachfolgenden Tabelle 3 zusammengefaßt. Die Daten der Tabelle 3 sind außerdem auch noch in den Figuren 5 bis 9 graphisch veranschaulicht.

Die gezeigten Ergebnisse lassen bei allen gemessenen Prohormonen bei Sepsispatienten gegenüber Normalpersonen einen mehr oder weniger klar ausgeprägten Anstieg der Werte erkennen, wenn auch die Unterscheidung von Normalpersonen und Sepsispatienten bei der Bestimmung von Procalcitonin am ausgeprägtesten ist.

Bei einer ergänzenden Literaturrecherche wurde ferner eine Veröffentlichung in J. Endocr. (1988) 119, S.159-165 ermittelt, die sich mit der Charakterisierung von Pro-Opiomelanocortin-(POMC)-verwandten Peptiden bei septischem Schock befaßte. Es geht in der Veröffentlichung um die Frage einer erhöhten endogenen Opioid-Aktivität bei septischem Schock und deren Beeinflussung durch Verabreichung von Steroiden. Ein direkter Einfluß eines infektiösen Geschehens oder eine Beeinflussung der Meßwerte durch Antibiotika wird nicht diskutiert. Aufgrund der Fragestellung in der genannten Veröffentlichung besteht keine logische Möglichkeit zu einer verallgemeinernde Diskussion der berichteten Ergebnisse unter Einbeziehung anderer Prohormon-Peptide. Erst angesichts der hierin offenbarten Lehre der vorliegenden Erfindung läßt sich die genannte Veröffentlichung retrospektiv als weiterer Hinweis auf eine generelle Erhöhung von Prohormonen bei Sepsis interpretieren.

**Tabelle 3:**

| | PCT | Pro-ANF | Pro-ADM | Pro-END | Pro-BNP |
|---|---|---|---|---|---|
| | [ng/ml] | [pg/tube] | [pg/tube] | [pg/tube] | [pg/tube] |
| Normalpatienten: | | | | | |
| **A** | 0,07 | 69,4 | 64 | 29,3 | 31,1 |
| **B** | 0,26 | 26,6 | 50,2 | 15,4 | 30,1 |
| **C** | 0,10 | 14,7 | 1,0 | 1,0 | 24,7 |
| **D** | 0,06 | 53,2 | 77,8 | 19,3 | 27,4 |
| **E** | 0,06 | 51,1 | 66,5 | 20,6 | 25,4 |
| **F** | 0,04 | 95,5 | 53,5 | 28,2 | 28,9 |
| **G** | 0,07 | 117 | 83,5 | 18,3 | 17,3 |
| **H** | 0,10 | 88,1 | 52,3 | 25,1 | 28,1 |
| **I** | 0,10 | 69,4 | 107 | 23,2 | 25 |
| **J** | 0,07 | 38,3 | 91,1 | 26,1 | 25,7 |
| **K** | 0,06 | 111 | 64,9 | 22,8 | 29,6 |
| **L** | 0,09 | 73,8 | 66,3 | 32,4 | 21,6 |
| **M** | 0,07 | 42 | 58,9 | 27,1 | 23,7 |
| **N** | 0,09 | 107 | 114 | 27,3 | 31,2 |
| **O** | 0,10 | 56,7 | 62,5 | 20,3 | 26,2 |
| **P** | 0,10 | 47,2 | 51,7 | 24,5 | 33,3 |
| **Q** | 0,12 | 155 | 72,5 | 34,6 | 36,7 |
| **R** | 0,13 | 92,1 | 64,7 | 29,6 | 35,2 |
| **S** | 0,12 | 153 | 100 | 34,7 | 36 |
| **T** | 0,11 | 78 | 69,6 | 27,1 | 37,7 |
| Sepsispatienten : | | | | | |
| 13a | 1,1 | 333 | 195 | 70 | 34,9 |
| 10a | 11,9 | 62,5 | 154 | 30,5 | 30,4 |
| 18b | 6,4 | 323 | 209 | 75,4 | 46,4 |
| 19b | 21,2 | 346 | 180 | 71,6 | 50,7 |
| 8a | 1,8 | 303 | 203 | 70,3 | 48,8 |
| 9a | 24,7 | 271 | 205 | 78,4 | 33,6 |
| 9b | 29,0 | 305 | 210 | 62,5 | 40,3 |
| 12b | 5,8 | 324 | 204 | 67,4 | 36,1 |
| 10b | 1,9 | 127 | 128 | 30,5 | 33,9 |
| 16a | 86,7 | 347 | 198 | 83,3 | 39 |
| 8b | 1,1 | 138 | 153 | 29,6 | 33,7 |
| 20a | 1,4 | 167 | 176 | 41,3 | 30,9 |
| 20b | 1,1 | 170 | 178 | 39 | 34,9 |
| 13b | 0,8 | 295 | 186 | 45,3 | 36,9 |
| 19a | 17,6 | 354 | 201 | 58,6 | 54,5 |
| 7b | 2,5 | 356 | 199 | 78,6 | |
| 7a | 2,9 | 345 | 197 | 148 | |
| 16b | 40,0 | 343 | 197 | 88,1 | 43,9 |
| 12a | 5.2 | 327 | 216 | 76,1 | 34,3 |
| 15b | 1,9 | 420 | 215 | 82,5 | 52 |

## Patentansprüche

1. Verfahren zur Diagnose von Sepsis und sepsisähnlichen systemischen Infektionen, wobei Procalcitonin 3-116 in einer Probe aus dem Blut eines Patienten detektiert wird.

2. Verfahren nach Anspruch 1, wobei die Probe aus dem Blut eine Serumprobe ist.

3. Verfahren nach Anspruch 1, wobei die Probe aus dem Blut eine Plasmaprobe ist.

## Claims

1. Method for diagnosis of sepsis and sepsis-like systemic infections, wherein Procalcitonin 3-116 is detected in a sample from the blood of a patient.

2. Method according to claim 1, wherein the sample from the blood is serum sample.

3. Method according to claim 1, wherein the sample from the blood is plasma sample.

## Revendications

1. Procédé de diagnostic de septicémie et d'infections systémiques similaires, dans lequel on détecte la procalcitonine 3-116 dans un échantillon provenant du sang d'un patient.

2. Procédé selon la revendication 1, dans lequel l'échantillon provenant du sang est un échantillon de sérum.

3. Procédé selon la revendication 1, dans lequel l'échantillon provenant du sang est un échantillon de plasma.
